(19) Europäisches Patentamt
European Patent Office
Office européen des brevets

(11) Veröffentlichungsnummer: **0 313 945 A2**

# (12) EUROPÄISCHE PATENTANMELDUNG

(21) Anmeldenummer: 88117181.3

(22) Anmeldetag: 15.10.88

(51) Int. Cl.4: **A61K 31/55**

(30) Priorität: 30.10.87 DE 3736866

(43) Veröffentlichungstag der Anmeldung: 03.05.89 Patentblatt 89/18

(84) Benannte Vertragsstaaten: AT BE CH DE FR GB IT LI LU NL SE

(71) Anmelder: Dr. Karl Thomae GmbH
Postfach 1755
D-7950 Biberach (Riss)(DE)

(72) Erfinder: Rose, Peter, Dr. med.
Amriswilstrasse 7
D-7950 Biberach 1(DE)

(54) **Mittel zur Behandlung des Bluthochdrucks und der Herzinsuffizienz.**

(57) Die Verwendung der Verbindung der Formel

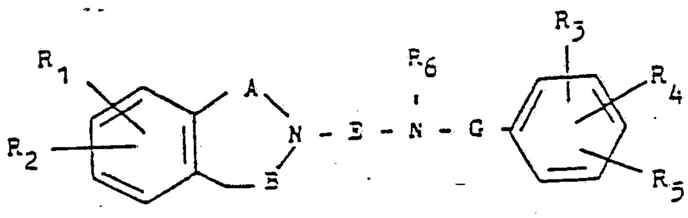

und deren physiologisch verträglichen Säureadditionssalze zur Behandlung des Bluthochdrucks und der Herzinsuffizienz.

Tabelle 1

Blutdruck systolisch

| | Placebo | 2 x 2,5 mg UL-FS 49 CL | 2 x 7,5 mg UL-FS 49 CL |
|---|---|---|---|
| Tag - 7 | 148 ± 18 | 157 ± 10 | 154 ± 14 |
| Tag + 1 | 147 ± 13 | 145 ± 18 | 146 ± 16 |
| Tag + 28 | 150 ± 14 | 142 ± 16 | 137 ± 13 |

EP 0 313 945 A2

Tabelle 2

Blutdruck diastolisch

| | Placebo | 2 x 2,5 mg UL-FS 49 CL | 2 x 7,5 mg UL-FS 49 CL |
|---|---|---|---|
| Tag - 7 | 91 ± 7 | 95 ± 8 | 93 ± 8 |
| Tag + 1 | 90 ± 7 | 90 ± 6 | 91 ± 7 |
| Tag + 28 | 89 ± 8 | 86 ± 6 | 86 ± 6 |

## Mittel zur Behandlung des Bluthochdrucks und der Herzinsuffizienz

In der EP-B-0.065.229 wird die Verbindung der Formel

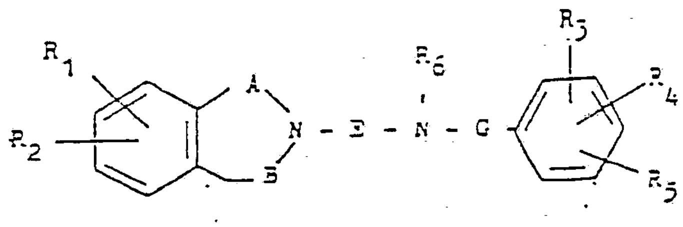

(I),

in der A eine $-CH_2-CH_2-$, $-CH=CH-$, - N H-CO-, - C $H_2$-CO- der
5 5

R7
-C =N-Gruppe
5

in der $R_7$ eine Alkylgruppe mit 1 bis 3 kohlenstoffatomen darstellt, und B die Methylen- oder Carbonylgruppe oder
A die -CO-CO-Gruppe und B die Methylengruppe,
E eine gegebenenfalls durch eine Alkylgruppe mit 1 bis 3 Kohlenstoffatomen substitutierte Ethylen-, n-Propylen-oder n-Butylengruppe, eine durch eine Hydroxygruppe in 2-Stellung substituierte n-Propylengruppe oder eine durch eine Hydroxygruppe in 2- oder 3-Stellung substituierte n-Butylengruppe,
G eine gegebenenfalls durch eine Alkylgruppe mit 1 bis 3 Kohlenstoffatomen substituierte Methylen- oder Ethylengruppe,
$R_1$ und $R_2$, die gleich oder verschieden sein können, Hydroxygruppen, Alkyl-, Alkoxy- oder Phenylalkoxygruppen, in denen der Alkylteil jeweils 1 bis 3 Kohlenstoffatome enthalten kann, oder einer der Reste $R_1$ oder $R_2$ auch ein Wasserstoffatom oder $R_1$ zusammen mit $R_2$ eine Alkylendioxygruppe mit 1 oder 2 Kohlenstoffatomen,
$R_3$ und $R_4$, die gleich oder verschieden sein können, Wasserstoff- oder Halogenatome, Hydroxygruppen, Alkyl- oder Alkoxygruppen mit jeweils 1 bis 4 Kohlenstoffatomen, Trifluormethyl- oder Cyangruppen oder einer der Reste $R_3$ oder $R_4$ auch eine Nitrogruppe oder $R_3$ zusammen mit $R_4$ eine Alkylendioxygruppe mit 1 oder 2 Kohlenstoffatomen,
$R_5$ ein Wasserstoffatom, eine Alkyl-, Hydroxy-, Alkoxy-, Amino-, Alkylamino-, Dialkylamino-, Alkanoylamino-, Alkoxycarbonylamino- oder Bis(alkoxycarbonyl)aminogruppe, in denen der Alkylteil jeweils 1 bis 3 Kohlenstoffatome enthalten kann, oder eine durch eine Trifluormethylgruppe substitutierte Methyalmino- oder Ethylaminogruppe, und
$R_6$ ein Wasserstoffatom, eine Alkyl, Phenylalkyl, Alkanoyl- oder Alkoxycarbonylgruppe, in denen der Alkylteil jeweils 1 bis 3 Kohlenstoffatome enthalten kann, oder eine Alkylengruppe mit 3 bis 5 Kohlenstoffatomen bedeuten, und deren Säureadditionssalze, insbesondere deren physiologisch verträgliche Säureadditionssalze mit anorganischen oder organischen Säuren, beschrieben, wobei festgestellt wird, daß dieser Verbindung und deren Säureadditionssalzen wertvolle pharmakologische Eigenschaften zukommen, z.B. eine lang anhaltende herzfrequenzsenkende Wirkung sowie eine Herabsetzung des $0_2$-Bedarfs des Herzens.

Diese Verbindungen wurden daher als neuartige Substanzen entwickelt, welche die Herzfrequenz über einen direkten Angriff am Sinusknoten senken. Hierdurch kommen den Substanzen antiischämische Eigenschaften zu, d.h. neben einer Senkung des myokardialen Energieverbrauches durch die Herzfrequenz wird gleichzeitig das Sauerstoffangebot über eine Verlängerung der Diastole erhöht. Pharmakologischen Untersuchungen zufolge beeinflussen die Substanzen weder die Inotropie noch den Blutdruck oder die AV-Überleitung. Tierexperimentell konnte ein zusätzlicher sog. myokardprotektiver Effekt nachgewiesen wurden. Somit erscheinen die Substanzen geeignet für die Therapie der stabilen koronaren Herzkrankheit.

Überraschenderweise wurde nun bei Prüfung am Menschen festgestellt, daß sie wertvolle blutdrucksenkende Eigenschaften besitzen.

Gegenstand der Erfindung ist daher die Verwendung der Verbindungen der allgemeinen Formel I und deren Säureadditionssalze, insbesondere deren physiologisch verträglichen Säure additionssalze mit anorganischen oder organischen Säure, zur Behandlung von Bluthochdruck und/oder Herzinsuffizienz.

Weiterer Gegenstand der Erfindung ist die Verwendung dieser Verbindungen und deren Säureadditionssalze zur Herstellung eines pharmazeutischen Präparates zur Behandlung von Bluthochdruck und/oder Herzinsuffizienz.

Vorzugsweise verwendet man 1,3,4,5-tetrahydro-7,8-dimethoxy-3-[3-[[2-(3,4-dimethoxyphenyl)ethyl]-methyl]amino]propyl]-2H-3-benzazepin-2-on und deren physiologisch verträglichen Säureadditionssalze, insbesondere deren Hydrochlorid, das im folgenden als UL-FS 49 CL bezeichnet wird.

Es wird eine Tagesdosis von zweimal täglich 0.015 bis 0.2 mg/kg Körpergewicht, vorzugsweise 0.0175 mg/kg bis 0.175 mg/kg Körpergewicht erwartet, z.B. eine erwartende therapeutische Dosierung zur Senkung des arteriellen Blutdrucks wird sich vermutlich zwischen 1.25 und 7.5 mg/Mensch oral zweimal täglich bewegen. Die genaue Dosis wird offensichtlich vom Zustand des Patienten und der entsprechenden Prognose der Krankheit abhängen und könnte von der.oben angegeben Dosierung abweichen.

Zur medizinischen Anwendung läßt sich das Arzneimittel mit Hilfe von üblichen galenischen Hilfsstoffen wie z.B. Milchzucker, Mannit, Rohrzucker, mikrokristalline Zellulose, Magnesiumstearat, Polyvinylpyrollidon, Zitronensäure, Weinsäure, Wasser, Wasser/Äthanol, Wasser/Glycerin, Wasser/Sorbit, Wasser/Polyethylenglykol, Propylenglykol, Carboxymethylcellulose oder fetthaltigen Substanzen wie Hartfett oder deren geeigneten Gemischen in den üblichen Zubereitungen wie Tabletten, Dragées, Kapseln, Pulver Suspensionen, Tropfen, Ampullen, Säfte oder Zäpfchen herstellen.

Die Wirkung der Verbindungen wurde wie folgt festgestellt.

In einer doppelblinden, placebokontrollierten klinischen Prüfung des antianginösen Effekts von UL-FS 49 CL bei Patienten mit koronarer Herzkrankheit wurden insgesamt 60 Patienten in einer Studie aufgenommen. Alle Patienten waren bei Studienbeginn unbehandelt. In der 1. Woche (Tag -7 bis Tag -1) erhielten alle 60 Patienten Placebo. Während den darauffolgenden 4 Wochen wurden die 60 Patienten in 3 Gruppen zu je 20 Patienten eingeteilt. Die 1. Gruppe erhielt während dieser Zeit weiterhin Placebo. Die 2. Gruppe 2x 2.5 mg des Wirkstoffs und die 3. Gruppe 2x 7.5 mg des Wirkstoffs oral täglich (siehe Abb. 1).

An den Tagen -7, +1 und +28 wurden folgende Untersuchungen durchgeführt:

Klinische Untersuchung, Labor sowie EKG, Herzfrequenz und Blutdruck in Ruhe und unter Belastung.

In einer Zwischenaufwertung waren die vollständigen Daten von 48 Patienten verwendbar. Neben der Senkung der Herzfrequenz und Erhöhung der Belastungsdauer konnte überraschenderweise eine deutliche Blutdrucksenkung festgestellt werden. Die nachfolgenden Daten beziehen sich jeweils auf die Zeitpunkte 2 Stunden nach Applikation.

In der Placebo-Gruppe (n=17) blieben sowohl der systolische als auch der diastolische Blutdruck praktisch unverändert (Tab. 1, 2 und Abb. 2, 3). In der 2.5-mg-Gruppe (n=15) fiel der systolische Blutdruck von 157 ± 10 mm Hg (Tag -7) auf 142 ± 16 mm Hg (Tag +28) mit $p<0.001$ (Tab. 1, Abb. 2). Im gleichen Zeitraum fiel der diastolische Blutdruck von 95 ± 8 mm Hg auf 86 ± 6 mm Hg mit ebenfalls $p<0.001$ (Tab. 2, Abb. 3). In der 7.5-mg-Gruppe (n=16) wurde der systolische Blutdruck von 154 ± 14 mm HG (Tag -7) auf 137 ± 13 mm Hg (Tag +28) mit $p<0.001$ gesenkt (Tab. 1, Abb. 2). Der diastolische Blutdruck reduzierte sich in dieser Behandlungsgruppe von 93 ± 8 mm Hg auf 86 ±6 mm Hg mit $p<0.01$ (Tab. 2, Abb. 3).

Weder pharmakologisch noch klinisch-pharmakologisch wurden bischer blutdrucksenkende Effekte von UL-FS 49 CL und deren Säureadditionssalzen beschrieben. Physiologisch gesehen ist bei einer Senkung der Herzfrequenz wenn überhaupt, dann mit einer Erhöhung des systolischen Blutdruckes und gleichzeitig geringer Absenkung des diastolischen Blutdruckes zu rechnen. Der arterielle Mitteldruck müßte hierbei jedoch unverändert bleiben.

In der vorliegenden Studie konnte eindeutig ein statistisch signifikanter und klinisch relevanter Abfall von systolischem und diastolischem Blutdruck nachgewiesen werden.

Auf Grund dieser neuen Befunde eignen sich die vorliegenden Verbindungen, insbesondere UL-FS 49 CL, zur Behandlung des Bluthochdrucks und/oder der herzinsuffizienz.

Akute Toxizität

Die akute Toxizität von UL-FS 49 CL wurde an Mäusen, Ratten und Kaninchen nach den üblichen Methoden bestimmt. Aus dem Prozentsatz der Tiere, die nach den verschiedenen Dosen innerhalb von 14 Tagen verstarben, wurde nach Litchfield und Wilcoxon (J. Pharmacol. exp. Ther., 96, 99 (1949)) die $LD_{50}$ berechnet.

| Tierart | $LD_{50}$ mg/kg | |
|---|---|---|
| | oral | parenteral |
| Maus | 1.350 | i.v. 89 |
| Ratte | 479 | - |
| Kaninchen | - | i.v. 45 |

BEISPIEL 1

Filmtabletten zu 1,25 mg

A. Tablettenkern:

Zusammensetzung:

| | | |
|---|---|---|
| (01) | UL-FS 49 CL | 1.25 mg |
| (02) | Lactose x $H_2O$ | 30.00 mg |
| (03) | Maisstärke | 16.00 mg |
| (04) | Kollidon 25 | 2.50 mg |
| (05) | Magnesiumstearat | 0.25 mg |
| | | 50.00 mg |

Herstellung:

Der Wirkstoff wird mit 5 mg (02) gründlich verrieben. Die Verreibung wird mit (03) und dem Reste von (02) gemischt und mit einer wäßrigen Lösung von (04) gleichmässig befeuchtet. Die feuchte Masse wird durch ein Sieb von 1,5 mm Maschenweite gegeben, in einem Umluftschrank getrocknet, erneut gesiebt (1.0 mm Maschenweite) und mit (05) vermischt. Aus dieser Mischung werden bikonvex gewölbte Tabletten von 5 mm Durchmesser und 50 mg Gewicht auf einer Tablettiermaschine hergestellt.

B. Filmüberzug

Zusammensetzung:

| | | |
|---|---|---|
| (01) | Hydroxypropylmethylcellulose | 1.5 mg |
| (02) | Polyethylenglykol 6000 | 0.3 mg |

Herstellung;

Die Filmbestandteile (01) und (02) werden in Wasser gelöst und mit Hilfe einer Zweistoffdüse auf die Tablettenkerne aufgesprüht.

Beschreibung einer Filmtablette

| | |
|---|---|
| Aussehen: | weiß, rund, bikonvex |
| Durchmesser: | ca. 5.1 mm |
| Gewicht: | ca. 51.8 mg |

BEISPIEL 2

Drageés zu 1,25 mg

A. Drageékern

vgl. Beispiel 1 A.

B. Drageéüberzug

Erfolgt in üblicher Weise mit einer zuckerhaltigen Dragiersuspension in einem gebräuchlichen Dragierkessel.

Beschreibung eines Drageés

| | |
|---|---|
| Aussehen: | weiß, rund, bikonvex |
| Durchmesser: | ca. 6 mm |
| Gewicht: | ca. 70 mg |

BEISPIEL 3

Kapsel mit 7.5 mg UL-FS 49 CL

Zusammensetzung:

1 Kapsel enthält:

| | |
|---|---|
| UL-FS 49 CL | 7.5000 mg |
| Maisstärke getr. | 85.7500 mg |
| Lactose x $1H_2O$ | 85.7500 mg |
| Magnesiumstearat | 1.0000 mg |
| | 180.0000 mg |

Kapselhülle:

| | |
|---|---|
| Hartgelatine-Kapseln Nr. 3 | |
| lederfarben | 50.0000 mg |
| | 230.0000 mg |

Herstellungsverfahren:

Wirkstoff, Maisstärke und Lactose werden gemischt, mit Granulierflüssigkeit (Ethanol/Wasser 1 + 1 G/G) durchfeuchtet und gesiebt.

Nach Trocknung bei 50 °C wird das Granulat nochmals gesiebt und anschließend Magnesiumstearat zugegeben.

Das komplette Kapselgranulat wird homogen gemischt und auf einer geeigneten Kapselmaschine in Hartgelatinekapseln abgefüllt.

BEISPIEL 4

Ampullen mit 5 mg UL-FS 49 CL

Zusammensetzung: mg/5 ml

| | | |
|---|---|---|
| UL-FS 49 CL | | 5.000 mg |
| Citronensäure x $H_2O$ | | 2.000 mg |
| 1N NaOH ad pH 6.0 | ca. | 0.025 ml |
| NaCl | | 46.500 mg |
| $H_2O$ f. Injektionszwecke | ad | 5.000 ml |

Herstellungsverfahren:

In Wasser für Injektionszwecke werden Citronensäure, Wirkstoff und Natriumchlorid bei Raumtemperatur gelöst. In IN NaOH wird auf pH 6 eingestellt und mit Wasser für Injektionszwecke auf das Ansatzgewicht aufgefüllt. Nach Sterilfiltration wird die Lösung in Spießampullen abgefüllt. Die gefüllten Ampullen werden 20 Minuten bei 121 °C autoklaviert.

BEISPIEL 5

Suppositorien mit 10 mg UL-FS 49 CL

Zusammensetzung:

| | |
|---|---|
| UL-FS 49 CL | 0.010 g |
| Hartfett (z.B. Witepsol H 19 und Witepsol W 45) | 1.690 g |
| | 1.700 g |

Herstellungsverfahren:

Das Hartfett wird geschmolzen. Bei 38° C wird die gemahlene Wirksubstanz in der Schmelze homogen dispergiert. Es wird auf 35° C abgekühlt und in schwach vorgeKühlte Suppositorienformen ausgegossen.

Ein Zäpfchen zu 1.7 g enthält 10 mg UL-FS 49 CL.

BEISPIEL 6

Tropfenlösung, enthaltend 5 mg/l ml/20 Tropfen UL-FS 49 CL

Zusammensetzung:

| | | |
|---|---|---|
| UL-FS 49 CL | | 0.50 g |
| Hydroxyethylcellulose | | 0.15 g |
| Weinsäure | | 0.10 g |
| Sorbitlösung 70 % Trockensubstanz | | 30.00 g |
| Glycerin | | 10.00 g |
| Benzoesäure | | 0.15 g |
| Dest. Wasser | ad | 100.00 ml |

Herstellungsverfahren:

Dest. Wasser wird auf 70° C erhitzt. Hierin wird unter Rühren Hydroxyethylcellulose, Benzoesäure und Weinsäure gelöst. Es wird auf Raumtemperatur abgekühlt und hierbei das Glycerin und die Sorbitlösung unter Rühren zugegeben. Bei Raumtemperatur wird die Wirkstoff zugegeben und bis zur völligen Auflösung gerührt. Anschließend wird zur Entlüftung der Lösung unter Rühren evakuiert.

1 ml, entsprechend 20 Tropfen der Tropfenlösung, enthalten 5,0 mg UL-FS 49 CL.

BEISPIEL 7

Saft mit 2.5 mg UL-FS 49 CL / 5 ml

Zusammensetzung:

| | | |
|---|---|---|
| UL-FS 49 CL | | 0.05 g |
| Carboxymethylcellulose | | 0.10 g |
| p-Hydroxybenzoesäuremethylester | | 0.05 g |
| p-Hydroxybenzoesäurepropylester | | 0.03 g |
| Saccarose | | 10.00 g |
| Glycerin | | 5.00 g |
| Sorbitlösung 70 % | | 20.00 g |
| Aroma | | 0.30 g |
| Wasser, dest. | ad | 100.00 ml |

Herstellungsverfahren:

Dest. Wasser wird auf 70 °C erhitzt. Hierin wird unter Rühren p-Hydroxybenzoesäuremethylester und -propylester sowie Glycerin und Carboxymethylcellulose gelöst. Es wird auf Raumtemperatur abgekühlt und unter Rühren der Wirkstoff zugegeben und gelöst. Nach Zugabe und Lösung der Saccarose, der Sorbitlösung und des Aromas wird der Saft zur Entlüftung unter Rühren evakuiert.

5 ml Saft enthalten 2,5 mg UL-FS 49 Cl.

**Ansprüche**

1. Die Verwendung einer Verbindung der allgemeinen Formel

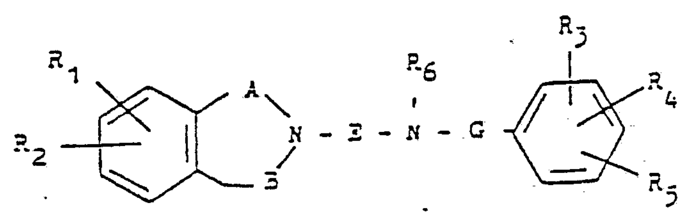

(I),

in der A eine $-CH_2-CH_2-$, $-CH=CH-$, - NH-CO-, - CH2-CO- oder

R7
-C =N-Gruppe

in der $R_7$ eine Alkylgruppe mit 1 bis 3 Kohlenstoffatomen darstellt, und B die Methylen- oder Carbonylgruppe oder

A die -CO-CO-Gruppe und B die Methylengruppe, E Eine gegebenenfalls durch eine Alkylgruppe mit 1 bis 3 Kohlenstoffatomen substitutierte Ethylen-, n-Propylen- oder n-Butylengruppe, eine durch eine Hydroxygruppe in 2-Stellung substituierte n-Propylengruppe oder eine durch eine Hydroxygruppe in 2- oder 3-Stellung substituierte n-Butylengruppe,

G eine gegebenenfalls durch eine Alkylgruppe mit 1 bis 3 Kohlenstoffatomen substituierte Methylen- oder Ethylengruppe,

$R_1$ und $R_2$, die gleich oder verschieden sein können, Hydroxygruppen, Alkyl, Alkoxy- oder Phenylalkoxygruppen, in denen der Alkylteil jeweils 1 bis 3 Kohlenstoffatome enthalten kann, oder einer der Reste $R_1$ oder $R_2$ auch ein Wasserstoffatom oder $R_1$ zusammen mit $R_2$ eine Alkylendioxygruppe mit 1 oder 2 kohlenstoffatomen, $R_3$ und $R_4$, die gleich oder verschieden sein können, Wasserstoff- oder Halogenatome, Hydroxygruppen, Alkyl- oder Alkoxygruppen mit jeweils 1 bis 4 Kohlenstoffatomen, Trifluormethyl- oder Cyangruppen oder einer der Reste $R_3$ oder $R_4$ auch eine Nitrogruppe oder $R_3$ zusammen mit $R_4$ eine Alkylendioxygruppe mit 1 oder 2 Kohlenstoffatomen,

$R_5$ ein Wasserstoffatom, eine Alkyl-, Hydroxy-, Alkoxy-, Amino-, Alkylamino-, Dialkylamino-, Alkanoylamino-, Alkoxycarbonylamino- oder Bis(alkoxycarbonyl) aminogruppe, in denen der Alkylteil jeweils 1 bis 3 Kohlenstoffatome enthalten kann, oder eine durch eine Trifluormethylgruppe substitutierte methyalmino- oder Ethylaminogruppe, und

$R_6$ ein Wasserstoffatom, eine Alkyl-, Phenylalkyl, Alkanoyl- oder Alkoxycarbonylgruppe, in denen der Alkylteil jeweils 1 bis 3 Kohlenstoffatome enthalten kann, oder eine Alkylengruppe mit 3 bis 5 Kohlenstoffatomen bedeuten, und deren Säureadditionssalzen, insbesondere deren physiologisch verträgliche Säureadditionssalze mit anorganischen oder organischen Säuren, zur Herstellung eines pharmazeutischen Präparates zur Behandlung von Bluthochdruck und/oder Herzinsuffizienz.

2. Die Verwendung der Verbindung der Formel I wie in Anspruch 1 angegeben und deren physiologisch verträgliche Säureadditionssalze zur Behandlung von Bluthochdruck und/oder Herzinsuffizienz.

3. Die Verwendung gemäß Anspruch 1 oder 2, dadurch gekennzeichnet, daß man 1,3,4,5-tetrahydro-7,8-dimethoxy-3-[3-[[2-(3,4-dimethoxyphenyl)ethyl]methyl]amino]propyl]-2H-3-benzazepin-2-on oder eine seiner physiologisch verträglichen Säureadditionssalze verwendet.

4. Die Verwendung gemäß Anspruch 3, dadurch gekennzeichnet, daß als Säureadditionssalz das Hydrogenchlorid verwendet wird.

5. Ein pharmazeutisches Präparat zur Behandlung von Bluthochdruck und/oder Herzinsuffizienz, enthaltend 1.25 mg bis 7.5 mg 1,3,4,5-tetrahydro-7,8-dimethoxy-3-[3-[[2-(3,4-dimethoxyphenyl)ethyl]methyl]-amino]propyl]-2H-3-benzazepin-2-on oder eine seiner physiologisch verträglichen Säureadditionssalze.

ULFS 49 CL - Wirksamkeit bei KHK

(IP-Nr. 140.7)

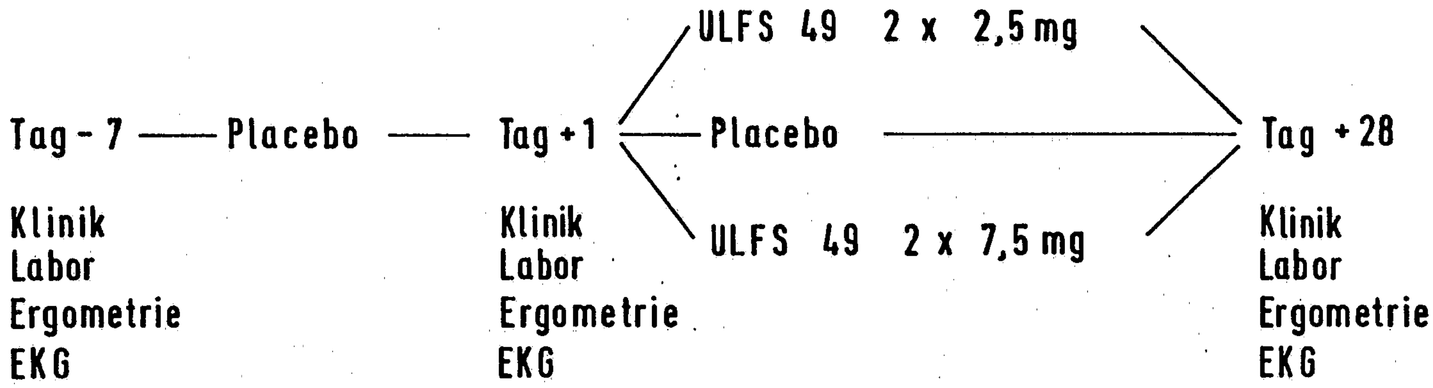


Fig. 1

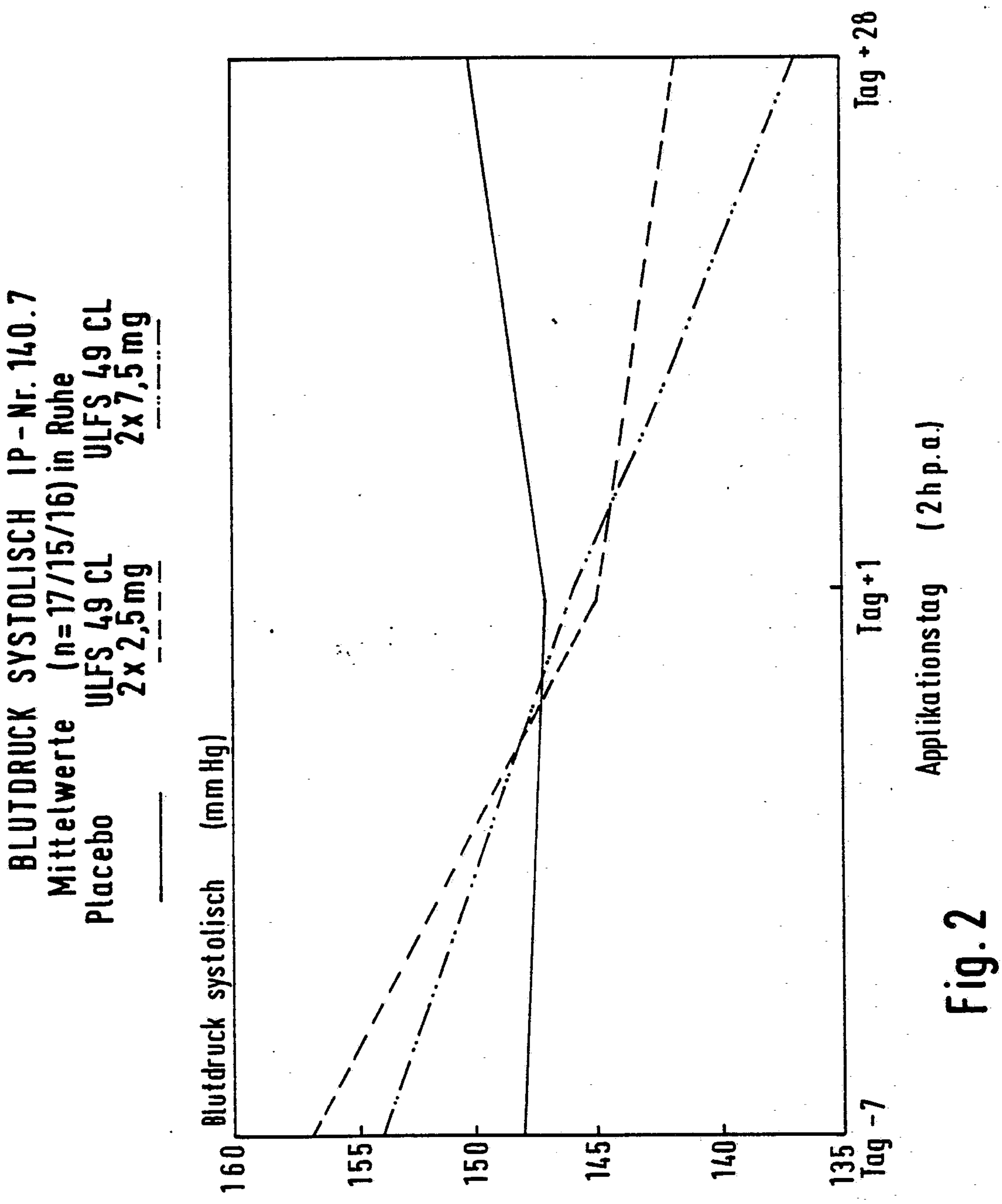
BLUTDRUCK SYSTOLISCH IP-Nr. 140.7
Mittelwerte (n=17/15/16) in Ruhe
Placebo
ULFS 49 CL 2x 2,5 mg
ULFS 49 CL 2x 7,5 mg
Blutdruck systolisch (mm Hg)
160
155
150
145
140
135
Tag -7
Tag +1
Tag +28
Applikationstag (2 h p.a.)

Fig. 2

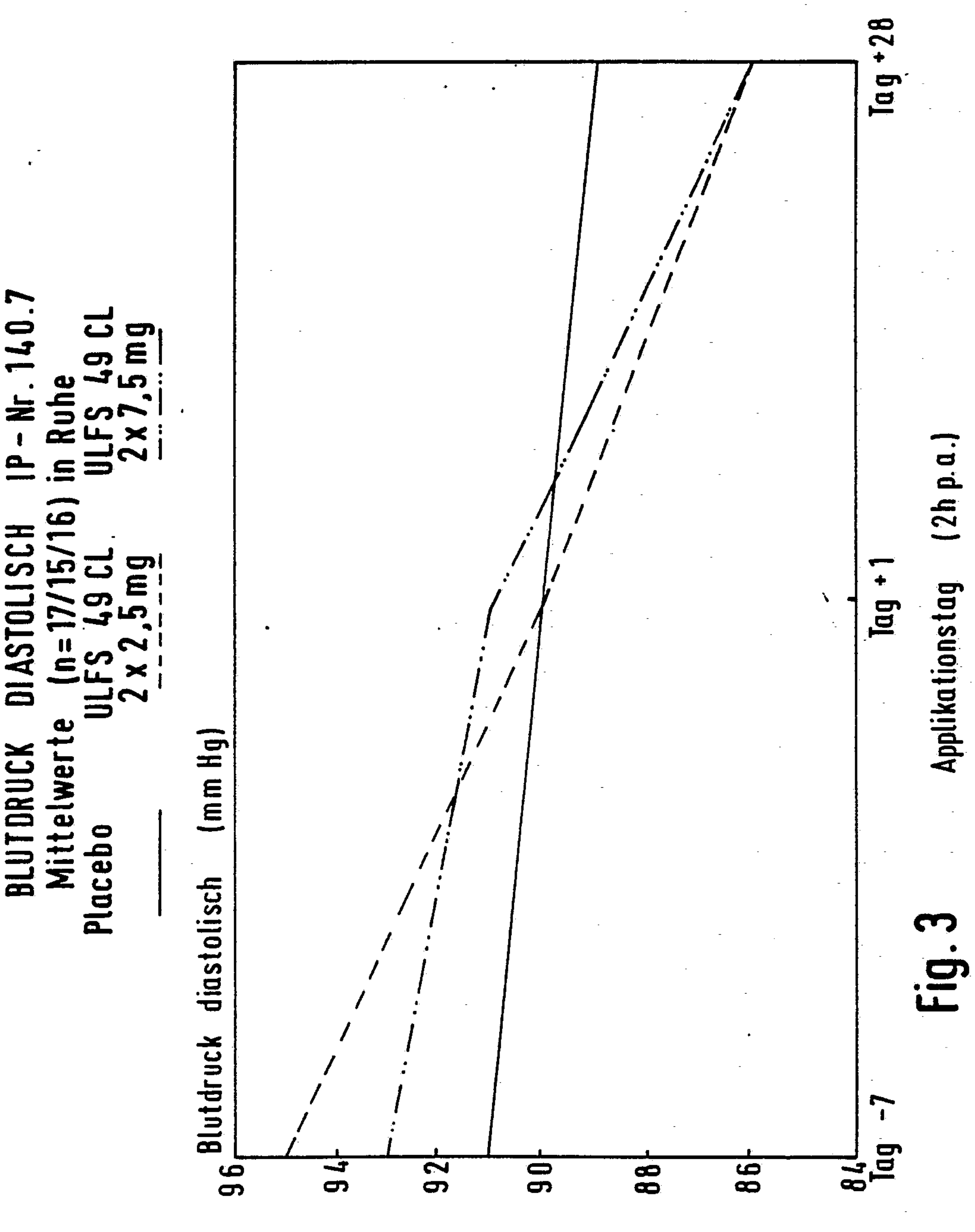
BLUTDRUCK DIASTOLISCH IP - Nr. 140.7
Mittelwerte (n=17/15/16) in Ruhe
Placebo
ULFS 49 CL
2 x 2,5 mg
ULFS 49 CL
2 x 7,5 mg
Blutdruck diastolisch (mm Hg)
96
94
92
90
88
86
84
Tag -7
Tag +1
Tag +28
Applikationstag (2h p.a.)

Fig. 3